# EUROPEAN PATENT APPLICATION

(11) **EP 2 708 553 A1**
(43) Date of publication of application: **19.03.2014**
(21) Application number: 12184878.2
(22) Date of filing: 18.09.2012
(51) Int. Cl.: C07K 14/37, C12N 15/31, C12N 15/63, C12N 1/14, C12P 21/00, C12R 1/885

(54) **Modified fungal cell**

(71) Applicant: Technische Universität Wien, 1040 Wien (AT)
(72) Inventor: Kubicek, Christian, 1100 Vienna (AT); Karimi, Razieh, 1180 Vienna (AT)
(74) Representative: Sonn & Partner Patentanwälte

(57) **Abstract**

The present invention relates to a genetically modified fungal cell of the genus Trichoderma capable to express the regulatory protein Vell in a higher amount compared to the unmodified wild type fungal cell.

SEQUENCE LISTING

<110>  TECHNISCHE UNIVERSITÄT WIEN

<120>  Modified fungal cell

<130>  R 61869

<160>  11

<170>  PatentIn version 3.5

<210>  1
<211>  573
<212>  PRT
<213>  Trichoderma reesei

<400>  1

Met Ala Thr Pro Ser Ser Val Ala Ser Ser Ser Ser Arg Asp Gln Val
1               5                   10                  15

Gln Arg Ile His Arg Val Thr Arg Glu Asn Arg His Leu Trp Tyr Gln
            20                  25                  30

Leu Thr Val Leu Gln Gln Pro Glu Arg Ala Arg Ala Cys Gly Ser Gly
        35                  40                  45

Met Lys Ala Asn Ser Asp Arg Arg Pro Val Asp Pro Pro Pro Val Val
    50                  55                  60

Glu Leu Arg Ile Ile Glu Gly Pro Ser Val Glu Glu Gly Lys Asp Ile
65                  70                  75                  80

Thr Phe Asp Tyr Asn Ala Asn Phe Phe Leu Tyr Ala Ser Leu Glu Gln
                85                  90                  95

Ala Arg Thr Ile Ala His Gly Arg Val Gln Asn Gly Ala Thr Asn Asn
            100                 105                 110

Pro Pro Ile Leu Thr Gly Val Pro Ala Ser Gly Met Ala Tyr Leu Asp
        115                 120                 125

Arg Pro Thr Glu Ala Gly Tyr Phe Ile Phe Pro Asp Leu Ser Val Arg
    130                 135                 140

His Glu Gly Tyr Phe Arg Leu Ser Phe Ser Leu Tyr Glu Thr Thr Lys
145                 150                 155                 160

Glu Pro Lys Asp Phe Asp Leu Glu Pro Ala Asp Ser Asp Leu Pro Pro
                165                 170                 175

Gly Val Asp Trp Arg Met Glu Ile Lys Thr Gln Pro Phe Asn Val Phe
            180                 185                 190

Ser Ala Lys Lys Phe Pro Gly Leu Met Glu Ser Thr Ser Leu Ser Lys
        195                 200                 205

Thr Val Ala Asp Gln Gly Cys Arg Val Arg Ile Arg Arg Asp Val Arg
    210                 215                 220

Met Arg Lys Arg Asp Gly Lys Gly Ser Gly Phe Asp Arg Arg Gly Glu
225                 230                 235                 240

Glu Glu Tyr Ser Arg Arg Arg Thr Val Thr Pro Ala Pro Ala Glu Asp
                245                 250                 255

Pro Asn Leu Arg Ala Arg Ser Val Ser Asn Ala Ser Glu His Arg Gly
            260                 265                 270

Pro Tyr Met Pro Gln Glu Pro Pro Arg Arg Pro Ser Ala Ala Glu Ser
        275                 280                 285

Tyr His Ala Pro Pro Pro Leu Pro Pro Pro Pro Pro Ser Ser Tyr Asp
    290                 295                 300

Ala Pro Pro Pro Ala Ala Arg Pro Gly His Leu Gly Phe Gly Gly Asp
305                 310                 315                 320

His Asn Met Pro Pro Gln Tyr Gly Ala Pro Ala Ala Arg Pro Tyr Gly
                325                 330                 335

His Pro Gln Ser Ala Pro Ile Pro Pro Ala Thr Pro Thr Gly Pro Tyr
            340                 345                 350

Pro Thr Ser Ser Ala Ala Pro Ser Pro Tyr Ala Lys Gln Asp His His
        355                 360                 365

His Gln Gln Gln Tyr Ser Tyr Asn Ser Arg Pro Pro Ala Pro Ser Ala
    370                 375                 380

Ser Pro Ala Pro Pro Met Lys His Ala Met Tyr Asp Asn Arg Pro Ser
385                 390                 395                 400

Glu Pro Tyr Val Pro His Ser Ser Pro Ser Val Tyr Ala Ser Thr Glu
                405                 410                 415

Arg Arg Pro Ser Tyr Ala Asn Tyr Ser Ala Pro Ala Pro Tyr Ser Thr
            420                 425                 430

Pro Ala Ser Gln Pro Thr Tyr Ser Thr Pro Ala Ser Gln Pro Thr Tyr
        435                 440                 445

Ser Thr Pro Ala Ser Gln Pro Thr Tyr Ser Ala Pro Pro Pro Ala Pro
    450                 455                 460

Tyr Ser Ala Pro Ala Pro Pro Pro Pro Arg Pro Ser Met Ser Gln Ser
465                 470                 475                 480

Ser Leu Ala Pro Leu Lys Ile Ala Ser Leu Val Ser Pro Leu Pro Pro
                485                 490                 495

Ile Glu Ala Gln Thr Glu Pro Leu Pro Pro Pro Pro Met Leu Ser Thr
            500                 505                 510

Gly Gly Lys Arg Lys His Asp His Val Phe Ser Gln Asn His Lys Pro
        515                 520                 525

Leu Tyr Asn Gly Gln Arg Gln Leu Asp Ala His Tyr Gly His Gly Tyr
    530                 535                 540

Arg Gly Leu Thr Pro Glu Pro Asp Gln Gly Leu Tyr Ser Arg Ala Asp
545                 550                 555                 560

Gly Gln Ile Gly Val Val Thr Phe Asn Gln Tyr Gln Val
                565                 570

<210>  2
<211>  1901
<212>  DNA
<213>  Trichoderma reesei

<400>  2
atggcgacgc cttcctccgt ggcctcgtcc agctcgcggg accaggtcca gcgcatccat      60

cgcgtgacga gagagaaccg tcacctttgg taccagttga cggttctaca gcagccggag     120

cgagcccgtg catgcggttc aggcatgaaa ggtgtgttgg ccactcgacg gaaccaagtg     180

aacaggcgat ggcgcgttgc aggccccccc ctttgctgac cctctcacag cgaacagtga     240

ccgacgacca gttgatccgc cgcctgttgt cgagctccgt atcatcgaag gtccttctgt     300

cgaggagggc aaggacatta ctttcgacta caatgccaac ttctttcttt atgccagtct     360

ggagcaggct cgcacgattg ctcacggccg cgtccaaaac ggcgccacca acaatcctcc     420

catcctgact ggtgttcctg catcgggcat ggcatacctc gaccggccaa ccgaggctgg     480

atatttcatc ttccccgacc tgtccgtccg acacgaaggc tacttccgcc tgtcctttag     540

cttgtacgag acgaccaagg agcccaagga ctttgaccta gagccggccg attccgacct     600

tccccctggg gtggactgga ggatggagat caagacgcag cctttcaacg tcttcagcgc     660

caagaagttc cctggactca tggagagtac ctcgctcagc aagaccgttg ccgaccaggg     720

ctgccgagtt cgaatccgcc gcgacgttcg catgaggaag cgcgacggca agggcagcgg     780

ctttgaccga cgcggtgagg aggagtactc tcgccgccga accgtgacgc cagctcccgc 840

cgaggatccc aacctgcgag ccagatccgt cagcaacgcg agcgagcacc gcgggccgta 900

catgccgcag gagccgccac ggcgaccatc cgctgccgag agctaccatg cccctccccc 960

actgcctcct ccgccgcctt cgtcgtacga cgctcctccg cctgccgctc gtcccggaca 1020

tctgggcttt ggcggcgatc acaacatgcc gccgcaatat ggcgcacccg ccgctcggcc 1080

atacggccac ccgcagagcg caccgatacc tcctgcgaca cccaccggac cctacccgac 1140

atcgtccgcg gcgccgtctc cgtatgccaa gcaggatcat catcatcagc agcagtacag 1200

ctacaactcc cgccctccgg cgcccagtgc ttccccagcg ccgccgatga agcacgccat 1260

gtacgacaac cggccatccg agccttatgt gcctcactcc tcgccttccg tatatgcgtc 1320

gaccgagagg cgaccttctt atgccaacta ctctgctcct gcgccctatt ctactcccgc 1380

ctcccagccg acctactcta ctcccgcctc ccagccgacc tactctactc ccgcctctca 1440

gccgacctac tcggctcccc cgccagcgcc atactcggct ccggcgcctc ctcctcctag 1500

gccgtcgatg tctcagtcgt ctctggcgcc gctcaagatt gcgtccctgg tgtctcccct 1560

gccgcccatt gaggctcaga ccgagcctct tcctccgcct cctatgctgt cgaccggtgg 1620

caagagaaaa catgaccacg tcttctccca gaaccacaag ccgctgtaca acggccagcg 1680

ccagcttgac gcgcactatg gccacggata tcgcgggctc acccccgagc ccgaccaggg 1740

cctatactct cgtgcggacg gtcaaatcgg cgttgtcacc ttcaaccaat accaggtgta 1800

aggccattgg acgagtgttt gtttgactcg gacccttggg tgacacggat tcactggagt 1860

atattatctg cattttttga tgtgtgatag atttctcatg g 1901

<210> 3
<211> 3017
<212> DNA
<213> Artificial Sequence

<220>
<223> tef1:vel1 construct

<400> 3
ggacagaatg tacagtacta tactacctac cttagatgcc atcacccctt cgcaggccat 60

ggaatttctg gcaagtcgca cccttcagcc atgcctgtcc gccatcatca taccagatcg 120

tgtcgtggcg ggcacttggg aaggacgagc tccaaccgcc gccgctgatt ggcctgatcc 180

gccacagcca ctggcgctcg cggcgaggaa aaaaaaaaat cttgcgggtg tgcagcagca 240

ttgagtggaa agcgggagcc gctgcacgac gctgattcgt ccgggctgcc tcgttagggc 300

tagaccagca ggcgggcgcc gcttattttg cccctcctca ggcttttttt cacaagcccc 360

gttttgctgc cccgcgcgtg gcagaaaaaa aaaatacagc acgagccact ggcacttgca 420

cccctccacc cctccgccga ccacctttta aatctcctcc tcccaccatc tgctttcgaa 480

attttctctc ttctgctctt cgtctcgcat acccggttca agcatccgat ctgcgaattt 540

gtatgtgctg cctctccctc tgaccttctg gtctggtgat accatcctcc ctcagtttgg 600

atcatcgcct tattcttctt ccctcttctg catctgcttc ctgctcgttt gaggaacatc 660

gccagctgac tctgcttgcc tcgcagcgat ctagtcaaga acaacacagc tctcacgcta 720

catcacacaa accgtcatcg atggcgacgc cttcctccgt ggcctcgtcc agctcgcggg 780

accaggtcca gcgcatccat cgcgtgacga gagagaaccg tcacctttgg taccagttga 840

cggttctaca gcagccggag cgagcccgtg catgcggttc aggcatgaaa ggtgtgttgg 900

ccactcgacg gaaccaagtg aacaggcgat ggcgcgttgc aggccccccc ctttgctgac 960

cctctcacag cgaacagtga ccgacgacca gttgatccgc cgcctgttgt cgagctccgt 1020

atcatcgaag gtccttctgt cgaggagggc aaggacatta ctttcgacta caatgccaac 1080

ttctttcttt atgccagtct ggagcaggct cgcacgattg ctcacggccg cgtccaaaac 1140

ggcgccacca acaatcctcc catcctgact ggtgttcctg catcgggcat ggcatacctc 1200

gaccggccaa ccgaggctgg atatttcatc ttccccgacc tgtccgtccg acacgaaggc 1260

tacttccgcc tgtcctttag cttgtacgag acgaccaagg agcccaagga ctttgaccta 1320

gagccggccg attccgacct tccccctggg gtggactgga ggatggagat caagacgcag 1380

cctttcaacg tcttcagcgc caagaagttc cctggactca tggagagtac ctcgctcagc 1440

aagaccgttg ccgaccaggg ctgccgagtt cgaatccgcc gcgacgttcg catgaggaag 1500

cgcgacggca agggcagcgg ctttgaccga cgcggtgagg aggagtactc tcgccgccga 1560

accgtgacgc cagctcccgc cgaggatccc aacctgcgag ccagatccgt cagcaacgcg 1620

agcgagcacc gcgggccgta catgccgcag gagccgccac ggcgaccatc cgctgccgag 1680

agctaccatg cccctccccc actgcctcct ccgccgcctt cgtcgtacga cgctcctccg 1740

cctgccgctc gtcccggaca tctgggcttt ggcggcgatc acaacatgcc gccgcaatat 1800

ggcgcacccg ccgctcggcc atacggccac ccgcagagcg caccgatacc tcctgcgaca 1860

cccaccggac cctacccgac atcgtccgcg gcgccgtctc cgtatgccaa gcaggatcat 1920

catcatcagc agcagtacag ctacaactcc cgccctccgg cgcccagtgc ttccccagcg 1980

ccgccgatga agcacgccat gtacgacaac cggccatccg agccttatgt gcctcactcc 2040

tcgccttccg tatatgcgtc gaccgagagg cgaccttctt atgccaacta ctctgctcct 2100

gcgccctatt ctactcccgc ctcccagccg acctactcta ctcccgcctc ccagccgacc 2160

tactctactc ccgcctctca gccgacctac tcggctcccc cgccagcgcc atactcggct 2220

ccggcgcctc ctcctcctag gccgtcgatg tctcagtcgt ctctggcgcc gctcaagatt 2280

gcgtccctgg tgtctcccct gccgcccatt gaggctcaga ccgagcctct tcctccgcct 2340

cctatgctgt cgaccggtgg caagagaaaa catgaccacg tcttctccca gaaccacaag 2400

ccgctgtaca acggccagcg ccagcttgac gcgcactatg gccacggata tcgcgggctc 2460

acccccgagc ccgaccaggg cctatactct cgtgcggacg gtcaaatcgg cgttgtcacc 2520

ttcaaccaat accaggtgta aggccattgg acgagtgttt gtttgactcg gacccttggg 2580

tgacacggat tcactggagt atattatctg cattttttga tgtgtgatag atttctcatg 2640

ggcgttttgg gaccagtacc cttgttcaaa atacctcttt tgttttctgt tggaagaggg 2700

aaatacgaag ggaacttttg cgggtattaa cggagtttgg gaacacatga caatagtttg 2760

agggaaattt ctttcattca tctttctctt ttccttatcc tttctttgtc tgttaaagtg 2820

agatggttca acggaacaca gcgacttcat tccaactctc ccctttgcat ctcattcttc 2880

tgttgtcttg ccatttttcg atatctttta tccgtgggca ccctttatgc gtggaatgct 2940

aatgagaggg acctgggata tggggagttt tctttctctt gccttttttt tttcttcctg 3000

aaagctttgg ggtacta 3017

<210> 4
<211> 27
<212> DNA
<213> Artificial Sequence

<220>
<223> Amplification primer

<400> 4
gctatcgatg gcgacgcctt cctccgt 27

<210> 5
<211> 22
<212> DNA
<213> Artificial Sequence

<220>
<223> Amplification primer

<400> 5
tagtacccca aagctttcag ga 22

<210> 6
<211> 21
<212> DNA
<213> Artificial Sequence

<220>
<223> Amplification primer

<400> 6
ccacattgcc tgcaagttcg c 21

<210> 7
<211> 22
<212> DNA
<213> Artificial Sequence

<220>
<223> Amplification primer

<400>  7
gtcggtgaaa gcctcaacgc ac                                                22


<210>  8
<211>  22
<212>  DNA
<213>  Artificial Sequence

<220>
<223>  Amplification primer

<400>  8
actacaacgg gtggaacatt ac                                                22


<210>  9
<211>  20
<212>  DNA
<213>  Artificial Sequence

<220>
<223>  Amplification primer

<400>  9
cgtggatgta cagcttctcg                                                   20


<210>  10
<211>  21
<212>  DNA
<213>  Artificial Sequence

<220>
<223>  Amplification primer

<400>  10
ccgagcttgg tagttactct g                                                 21


<210>  11
<211>  21
<212>  DNA
<213>  Artificial Sequence

<220>
<223>  Amplification primer

<400>  11
ggtagccttc ttgaactgag t                                                 21

## Description

The present invention relates to genetically modified fungi of the genus Trichoderma.

The plant cell wall consists of the β-(1,4)-linked glucose polymer cellulose, hemicellulose polysaccharides of varying composition, and lignin. The former two are formed by plants at an annual production rate of about 7.2 and 6 × 1010 tons, respectively. They thus represent the largest reservoir of renewable carbon sources on earth which could be used for the production of biofuels and other biorefinery products, thereby replacing products derived from fossile carbon components. While chemical and enzymatic processes for the hydrolysis of these polymers are known, enzymatic hydrolysis is preferred because it produces no inhibitory by-products and is thus environmentally compatible.

Trichoderma reesei is most widely used for the industrial production of cellulolytic and hemicellulolytic enzymes and has become a paradigm for research on these enzymes. Consequently, many details about the structure and function of these cellulases and hemicellulases have been elucidated, and several aspects of the regulation of their expression and secretion into the medium have also been described.

It is an object of the present invention to provide methods to increase the production of cellulolytic and hemicellulolytic enzymes and other enzymes subject to the same regulatory circuit such as carbohydrate esterases and chitinases in fungi of the Trichoderma genus. It is also an object of the present invention to provide modified fungi of the Trichoderma genus which show an increased production of cellulolytic and hemicellulolytic enzymes compared to their wild-type cells.

Therefore the present invention relates to a genetically modified fungal cell of the family Hypocreaceae, preferably of the genus Trichoderma or Hypocrea, capable to express the regulatory protein VEL1 in a higher amount compared to the unmodified wild type fungal cell.

Today, three transcriptional activators, i.e. XYR1, ACE2 and the HAP2/3/5 complex, as well as the two repressors CRE1 and ACE1 are known to be involved in cellulase regulation. One central regulator of developmental-specific gene expression in multicellular fungi, which has so far not been shown to affect cellulase and hemicellulase gene expression, is VELVET (VeA, VEL1). The velvet family of regulatory proteins is fungal specific, but highly conserved among ascomycetes and basidiomycetes. VeA was initially found in Aspergillus nidulans. Strains carrying a specific point mutation within said protein produced more conidia and fewer fruiting bodies than wild-type strains with an intact VeA protein. The role of VeA homologs in other ascomycetes is diverse, but always related to development and secondary metabolism. Recent studies have demonstrated the involvement of veA also in the secondary metabolism of different Aspergillus spp., Penicillium chrysogenum, Cephalosporium acremonium and Trichoderma virens (Bayram and Braus, FEMS Microbiol Rev. 36(2012): 1-24).

In 2008, it was found in A. nidulans that VeA (VEL1) and a second velvet family protein VelB form a trimeric complex with LaeA/LAE1 that is essential to coordinate secondary metabolism and development in darkness (Bayram et al., Science 320(2008): 1504-1506). However, a developmental regulation for cellulase and hemicellulase gene expression involving the VEL1 protein has not been described for Trichoderma or any other fungi.

It surprisingly turned out that the overexpression of VEL1 in fungi of the genus Trichoderma results in a significant increase of the production of cellulases, hemicellulases, carbohydrate esterases and/or other homo- and heteropolysaccharide degrading enzymes, preferably chitinases, within the cell. The increase in the expression of these proteins/enzymes is particularly advantageous because it allows, for instance, a better, faster and improved degradation of biological substrates comprising, e.g., cellulolytic substances. Furthermore, industrially relevant cellulases, hemicellulases, carbohydrate esterases and other homo- and heteropolysaccharide degrading enzymes such as chitinases can be produced much more efficiently than with wild type fungal cells.

The term "capable to express the regulatory protein VEL1 in a higher amount compared to the unmodified wild type fungal cell" as used herein means that the cells of the present invention are genetically modified in a manner that the cells are able to express Vel1 in a higher amount as an unmodified cell. The difference in the expression of VEL1 of a genetically modified and a wild type cell can be determined by the measurement of the mRNA transcripts using respective PCR techniques, by determining the amount of enzymes produced using methods based on enzyme specific antibodies (e.g. ELISA) or by determining the enzymatic activity of the overexpressed enzymes. Respective methods and means are well known in the art.

Methods for genetically modifying fungi, in particular fungi of the family Hypocreaceae, are well known to the person skilled in the art.

According to a preferred embodiment of the present invention the naturally occurring gene encoding the VEL1 protein is operably linked to a recombinant promoter capable to induce the increased expression of the VEL1 protein compared to the unmodified wild type fungal cell.

In order to overexpress VEL1 in the genetically modified fungal cell of the present invention the naturally occurring gene encoding the VEL1 protein of the fungal cell is operably linked to a recombinant promoter which is not the native Vel1 promoter of the wild type fungal cell. The exchange of the wildtype vel1 promoter allows increasing the expression rate of the VEL1 protein in the fungal cell provided of course that said recombinant promoter has a higher activity within the genetically modified fungal cell. Methods to introduce recombinantly promoters into specific sites of the chromosomal DNA of fungi, in particular of fungi of the genus Trichoderma, are well known to a person skilled in the art.

The conditions under which the VEL1 protein is overexpressed depend on the promoter used. If the promoter is an inducible promoter respective conditions or substances have to be applied in order to switch on or to regulate the promoter activity.

"Operably linked" refers to a juxtaposition wherein the components so described are in a relationship permitting them to function in their intended manner. A promoter "operably linked" to a coding sequence is present in the cell in such a way that expression of the coding sequence can be directly influenced by the promoter.

According to another preferred embodiment of the present invention the fungal cell comprises an expression vector carrying the gene encoding the VEL1 protein operably linked to a promoter.

In order to increase the expression rate of VEL1 in a host cell of the genus Trichoderma it is also possible to introduce into a host cell a vector carrying the gene encoding the VEL1 protein which is operably linked to a promoter. Since cells of the family Hypocreaceae, in particular of the genus Trichoderma, naturally express VEL1 said vector may comprise the wild type vell promoter operably linked to the vel1 gene. The presence of more than one copy of the vel1 gene under the control of the same promoter already results in an increased expression of VEL1 in such cells.

According to a preferred embodiment of the present invention the promoter is selected from the group consisting of *tef*1 promoter (transcription elongation factor 1), gpdl promoter, *pk*i*1* promoter, enol promoter, *pgk1* promoter and *cdna1* promoter (Uzbas F et al. Appl Microbiol Biotechnol 93(2012): 1601-1608; Trire2:110879, GenBank Accession No: GL985078.1; Trire2 accession number according to Martinez D, et al. Nat Biotechnol. 26(2008), 553-560).

Preferred promoters are also selected from the group of promoters consisting of *pki1*, *gpdA*, *gpd1* and hexl promoters. Further preferred promoters are also promoters disclosed, for instance, in Nakari-Setäla et al. (Appl. Env. Microbiol. 61 (1995), 3650-3655), Rahman et al. (Biosci. Biotechnol. Biochem. 73 (2009), 1083-1089; *egl3* and *xyn3* promoter), WO 98/23764 A1 (short *cbh1* promoter), EP 0 952 223 A1 (*cbh1*-promoter of Trichoderma viridae), US 7,393,664, US 7,517,685 (hex1 promoter) and Mach et al. (Curr. Genetics 25 (1994): 567-570; *pki1*). In a preferred embodiment of the present invention the promoters may be of heterologous or homologous origin.

The most preferred promoters are the *tef1* promoter, the *cdna1* promoter, the *pgk1* promoter and the *pki1* promoter.

Of course it is also possible to use any other promoter which can be used to regulate gene expression in host organisms such as Trichoderma reesei. Particularly preferred promoters are those regulating the protein expression of the following proteins in Trichoderma reesei: GH5 glycoside hydrolase (protein ID 81087), Bradorhizobium bleomycin resistance (protein ID 103009), unknown hypothetical protein (protein ID 106270), HHE domain protein, conserved (protein ID 70608), hypothetical conserved protein (protein ID 109925), PTH11-type GPCRs (protein ID 109146), MSF permease (protein ID 78585), glutathione-S-transferase (protein ID 112022), Catalase (protein ID 58472), mannose-6-phosphate isomerase (protein ID 60445), unknown protein (protein ID 105287), translation initiation regulator Gnc20 (protein ID 22839), imidazole proprionase-related amidohydrolase (protein ID 110757), MSF transporter (protein ID 3405), short chain dehydrogenase/reductase (protein ID 106164), MSF permease (protein ID 79202), unknown protein (protein ID 60370), hypothetical secreted protein (protein ID 122889), Flavonol reductase/cinnamoyl-CoA reductase (protein ID 111716), Zinc-binding oxidoreductase (protein ID 23292), hypothetical protein (protein ID 124198), unknown protein (protein ID 109523), Kynurenine aminotransferase, glutamine transaminase K (protein ID 122820), GPR1/FUN34/yaaH-like protein (protein ID 60810), hypothetical protein (protein ID 54352), Predicted Zn-dependent hydrolase (beta-lactamase superfamily) (protein ID 70197), sulfite transporter ssu1 (protein ID 2076), unknown protein (protein ID 4851), unknown secreted protein (protein ID 110830), unknown esterase/lipase (protein ID 70491) and MSF permease (protein ID 105260). These genes are regularly upregulated under cellulose inducing conditions (e.g. cultivation of Trichoderma reesei on lactose).

The fungal cell of the family Hypocreaceae is preferably a fungal cell of the genus Trichoderma or Hypocrea.

The Hypocreaceae are a family within the class Sordariomycetes. This family of fungi comprises several genera: Aphysiostroma, Cladobotryum, Gliocladium, Hypocrea, Hypocreopsis, Hypomyces, Mycogone, Nectria, Podostroma, Protocrea, Rogersonia, Sarawakus, Sepedonium, Sphaerostilbella, Sporophagomyces, Stephanoma and Trichoderma, whereby the fungal cell to be genetically modified according to the present invention is preferably of the genus Trichoderma or Hypocrea.

The Trichoderma genus comprises several species whereby Trichoderma reesei is the most preferred representative.

The amino acid sequence of the VEL1 protein and the nucleic acid sequence encoding the VEL1 protein can be identified and located within the genome of the members of the Hypocreaceae family using SEQ ID No. 1 and SEQ ID No. 2 disclosed herein. This information is enough to identify corresponding nucleic acid sequences and amino acid sequences in such cells by using methods known in the art (e.g. by applying PCR techniques, blot techniques etc).

According to a further preferred embodiment of the present invention said genetically modified fungal cell is capable to express at least 20%, preferably at least 50%, more preferably at least 100%, more of the regulatory protein VEL1 compared to the unmodified wild type fungal cell.

The genetically modified fungal cell of the present invention preferably comprises further a nucleic acid molecule comprising at least one promoter of a cellulose gene, hemicellulose gene, carbohydrate esterase gene and/or other genes of homo- and heteropolysaccharide degrading enzymes, preferably a chitinase gene, operably linked to a heterologous coding region.

The increased expression of the VEL1 protein in fungal cells of the family Hypocreaceae results in an increased expression of various proteins, in particular enzymes, which are involved in carbohydrate degradation processes. Such enzymes include cellulases (such as cellobiohydrolase I CEL7A, cellobiohydrolase II CEL6A, endo-β-1,4-glucanase EGL1), hemicellulases (such as enod-ß-1,4-xylanases and ß-xylosidases), carbohydrate esterases (such as acetyl xylan esterase AXE1) and/or other polysaccharide hydrolyzing enzymes such as exo-ß-1,4-glucanases, endo-chitinases). This clearly shows that the regulatory elements (in particular promoters) associated with these types of enzymes are influenced by the amount of VEL1 present in the cell. This relationship can be used to produce recombinant proteins in cells of the family Hypocreaceae by introducing nucleic acid molecules comprising at least one promoter of a cellulase gene, hemicellulase gene, carbohydrate esterase gene and/or other genes of homo- and heteropolysaccharide degrading enzymes, preferably of a chitinase gene, operably linked to a heterologous coding region.

"Heterologous coding region" as used herein refers to a coding region which is not naturally associated to promoters of a cellulase gene, hemicellulase gene, carbohydrate esterase gene and/or other genes of homo- and heteropolysaccharide degrading enzymes, preferably of a chitinase gene, present in cells of the family Hypocreaceae. These promoters are therefore operably linked to a nucleic acid stretch which may code for a protein or an enzyme which is not a cellulase, hemicellulase, carbohydrate esterase and/or other homo- and heteropolysaccharide degrading enzymes such as chitinase.

The term "cellulase" or "cellulolytic enzyme" refers to enzymes that hydrolyze a cellulosic material. Such enzymes include endoglucanases, cellobiohydrolases and beta-glucosidases. Enzymes belonging to this kind of enzymes can be identified by measuring their cellulolytic activity. Total cellulolytic activity is usually measured using insoluble substrates, including Whatman NQ1 filter paper, microcrystalline cellulose, bacterial cellulose, algal cellulose, cotton, pretreated lignocellulose, etc. The most common total cellulolytic activity assay is the filter paper assay using Whatman No 1 filter paper as the substrate. The assay was established by the International Union of Pure and Applied Chemistry (IUPAC) (Ghose, 1987, Measurement of cellulase activities, Pure Appl. Chem. 59: 257-68).

"Endoglucanases" include endo-1,4-(1,3;1,4)-beta-D-glucan 4-glucanohydrolase (E.C. 3.2.1.4), which catalyses endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. "Cellobiohydrolases" include 1,4-beta-D-glucan cellobiohydrolase (E.C. 3.2.1.91), which catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain. "Beta-glucosidases" include beta-D-glucoside glucohydrolase (E.C. 3.2.1.21), which catalyzes the hydrolysis of terminal non-reducing beta-D-glucose residues with the release of beta-D-glucose.

"Hemicellulases" or "hemicellulolytic enzymes" are enzymes that hydrolyze a hemicellulosic material. Hemicellulases are key components in the degradation of plant biomass. Examples of hemicellulases include, but are not limited to, an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates of these enzymes, the hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation.

According to a preferred embodiment of the present invention the heterologous coding region encodes a protein of interest.

A genetically modified fungal cell as described above can be used as a host for the recombinant expression of heterologous coding regions, in particular proteins and polypeptides. Therefore the heterologous coding region encodes preferably a protein, which according to the present invention includes also a polypeptide. This can include proteins from other microorganisms such as bacteria (e.g. *Clostridium* or archaebacterial carbohydrate hydrolases), yeast or fungi (e.g. laccase, cellobiose dehydrogenase), but also from higher eukaryotes such as calf chymosin, plasminogen activator, or antibody fragments (production of all these proteins by *Trichoderma* has been patented in the past).

According to another preferred embodiment of the present invention the at least one promoter of a cellulase gene, hemicellulase gene, carbohydrate esterase gene and/or gene of other homo- and heteropolysaccharide degrading enzymes such as chitinase is selected from the group consisting of genes that are particularly strongly induced by cellulosic substrates or by lactose such as the endo-ß-1,4-glucanase genes egl12 (Trire2:123232) or egl5 (Trire2:120312), the xylanase gene xyn2 (Trire2:123818), the GH16 endo-ß-1,3/1,4-glucanase Trire2: 55886, the GH27 α-galactosidase gene Trire2:72632, the cellobiohydrolae genes cbh1 (Trire2:123989) and cbh2 (Trire2:52567), the GH43 α-arabinofuranosidase gene Trire2: 3739, the endochitinase gene Trire2: 65162, or the acetyl-xylan esterase gene axel (Trire2: 73632), whereby the *cbh1* promoter is particularly preferred.

The nucleic acid molecule comprising at least one promoter of a cellulase gene, hemicellulase gene, carbohydrate esterase gene and/or gene of other homo- and heteropolysaccharide degrading enzymes such as chitinases gene operably linked to a heterologous coding region may also encode a signal sequence fused to the N-terminal portion of the encoded protein or polypeptide.

A "signal sequence" or "signal peptide" means a sequence of amino acids bound to the N-terminal portion of a protein which facilitates the secretion of the mature form of the protein outside the cells. The definition of a signal sequence is a functional one. The mature form of the extracellular protein lacks the signal sequence which is cleaved off during the secretion process. A signal sequence is operably linked to a protein or polypeptide if it directs the protein through the secretion system of a host cell. The signal sequences of *T. reesei cbh1* and *egl1* have particularly proven well for this purpose.

Another aspect of the present invention relates to a vector comprising a nucleic acid molecule encoding a regulatory protein VEL1 of a fungal cell of the family Hypocreaceae, preferably of the genus Trichoderma or Hypocrea, having at least 80%, preferably at least 90%, more preferably at least 95%, even more preferably at least 98%, in particular 100%, identity to SEQ ID No. 1 and a promoter operably linked to said nucleic acid molecule. The promoter operably linked to said nucleic acid molecule may be the wild-type *vell* promoter or any other promoter disclosed herein. This means that in the present patent application genetically modified fungal cells are provided which comprise more than one copy of the vel1 gene within the genome or within the cell.

The term "identity", as used herein, indicates whether any two (or more) peptide, polypeptide or protein sequences have amino acid sequences that are "identical" to a certain degree ("% identity") to each other. This degree can be determined using known computer algorithms such as the "FASTA" program, using for example, the default parameters as in Pearson et al. (1988) PNAS USA 85: 2444 (other programs include the GCG program package (Devereux, J., et al., Nucleic Acids Research (1984) Nucleic Acids Res., 12, 387-395), BLASTP, BLASTN, FASTA (Atschul, S.F., et al., J Molec Biol 215: 403 (1990); Guide to Huge Computers, Martin J. Bishop, ed., Academic Press, San Diego, 1994, and Carilloet al, (1988)SIAM J Applied Math 48 : 1073). For instance, the BLAST tool of the NCBI database can be used to determine identity. Other commercially or publicly available programs include, DNAStar "MegAlign" program (Madison,WI) and the University of Wisconsin Genetics Computer Group(UWG) "Gap" program (Madison, WI)). Percent identity of protein molecules can further be determined, for example, by comparing sequence information using a GAP computer program (e.g. Needleman et al., (1970) J. Mol. Biol. 48:443, as revised by Smith and Waterman (1981) Adv. Appl. Math. 2:482). Briefly, the GAP program defines identity as the number of aligned symbols (i.e., nucleotides or amino acids) which are identical, divided by the total number of symbols in the shorter one of the two sequences. Default parameters for the GAP program can include : (1) a unary comparison matrix (containing a value of 1 for identities and for non-identities) and the weighted comparison matrix of Gribskov et al. 14:6745, as described by Schwartz and Dayhoff, eds., ATLAS OF PROTEIN SEQUENCE AND STRUCTURE, National Biomedical Research Foundation, pp. 353-358 (1979); (2) a penalty of 3.0 for each gap and an additional 0.10 penalty for each symbol in each gap; and (3) no penalty for end gaps.

In order to transfer a nucleic acid molecule encoding the polypeptide of the present invention to a host cell the nucleic acid molecule of the present invention is provided in a vector. The vector may be an expression vector capable to express the polypeptide of the present invention. However, the vector of the present invention may also be a recombination vector which allows to transfer a nucleic acid molecule encoding the polypeptide of the present invention into the genome of a host cell. The vector of the present invention may comprise further elements such as additional coding sequences within the same transcription unit, controlling elements such as promoters, ribosome binding sites, transcription terminators, polyadenylation sites, additional transcription units under control of the same or different promoters, sequences that permit cloning, expression, homologous recombination, and transformation of a host cell. According to the present invention any vector can be used to modify a fungal cell of the family Hypocreaceae, preferably of the genus Trichoderma or Hypocrea, provided that the introduction of said vector into the cell results in an overexpression of the VEL1 protein compared to the wildtype cell.

It is particularly preferred that the promoter is not the native vel1 promoter, wherein the promoter is preferably selected from the group consisting of *tef1* promoter (transcription elongation factor 1), *gpd1* promoter, *pki1* promoter, enol promoter, *pgk1* promoter and *cdna1* promoter (Uzbas F et al. Appl Microbiol Biotechnol 93(2012): 1601-1608; Trire2:110879, GenBank Accession No: GL985078.1). Further preferred promoters are described above.

Yet another aspect of the present invention relates to a cell of the family Hypocreaceae, preferably of the genus Trichoderma or Hypocrea, in particular Trichoderma reesei, comprising a vector according to the present invention. Particularly preferred fungal cells are described above.

According to a particularly preferred embodiment of the present invention, the genetically modified fungal cell of the present invention comprises a tef1 promoter operably linked to vel1, preferably having nucleic acid sequence SEQ ID No. 3.

In order to increase the amount of VEL1 within the host cell more than one copy of the nucleic acid molecule or vector according to the present invention are preferably provided in said cells.

A further aspect of the present invention relates to a method for producing cellulases, hemicellulases, carbohydrate esterases and/or other homo- and heteropolysaccharide degrading enzymes such as chitinases in a fungal cell of the family Hypocreaceae, preferably of the genus Trichoderma or Hypocrea, comprising the steps of:
a) providing a wild type fungal cell of the family Hypocreaceae or a mutated variant therof,
b) genetically modifying said wild type fungal cell by replacing the promoter of the gene encoding the regulatory protein VEL1 by a promoter capable to express the regulatory protein VEL1 in a higher amount compared to the unmodified wild type fungal cell (see above) or introducing additional copies of the vel1 gene into the genome or by introducing a vector according to the present invention,
c) cultivating the genetically modified fungal cell of the family Hypocreaceae of step b) in a cultivation medium, and
d) isolating the cellulases, hemicellulases, carbohydrate esterases and/or other homo- and heteropolysaccharide degrading enzymes such as chitinases produced by said fungal cell.

The genetically modified fungi of the family Hypocreaceae described herein can be used for the production of proteins having the same regulatory circuit as cellulases, hemicellulases, carbohydrate esterases and/or other homo- and heteropolysaccharide degrading enzymes such as chitinases. It turned surprisingly out that the overproduction of the VEL1 protein within a fungal cell of the family Hypocreaceae (i.e. higher expression of VEL1 compared to the unmodified wild-type fungal cell) results in an overproduction of cellulases, hemicellulases, carbohydrate esterases and/or other homo- and heteropolysaccharide degrading enzymes such as chitinases in said fungal cell. This increase of the protein production can be used in a method for producing cellulases, hemicellulases, carbohydrate esterases and/or other homo- and heteropolysaccharide degrading enzymes such as chitinases. These proteins are industrially used in various areas such as the production of biofuel.

According to a preferred embodiment of the present invention the cultivation medium in which the genetically modified fungal cell is cultivated comprises cellulosic or hemicellulosic carbohydrates or lactose. Such substrates are known to induce, e.g. the expression of cellulases in fungi of the family Hypocreaceae. Appropriate cellulosic or hemicellulosic carbohydrates used to grow fungi of the family Hypocreaceae are known in the art.

The cellulosic and/or hemicellulosic material comprising cellulosic and/or hemicellulosic carbohydrates can be any material containing cellulose and/or hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in the stems, leaves, hulls, husks, and cobs of plants or leaves, branches, and wood of trees. The cellulosic material can be, but is not limited to, herbaceous material, agricultural residue, forestry residue, municipal solid waste, waste paper, and pulp and paper mill residue. It is understood herein that the cellulose may be in the form of lignocellulose, a plant cell wall material containing lignin, cellulose, and hemicellulose in a mixed matrix. In a preferred aspect, the cellulosic material is lignocelluloses, which comprises cellulose, hemicellulose, and lignin.

Other cellulosic material include corn fiber, corn cob, orange peel, rice straw, wheat straw, microcrystalline cellulose, bacterial cellulose, algal cellulose, cotton linter, amorphous phosphoric-acid treated cellulose, filter paper etc.

The cellulosic material used as substrate in the medium may be used as is or may be subjected to pretreatment, using conventional methods known in the art.

Conditions under which the genetically modified fungi of the family Hypocreaceae can be cultivated are known in the art.

The cultivation medium in which the fungi of the family Hypocreaceae are cultivated can be a liquid medium or a (semi)solid medium. In the submerged (fed-)batch process, T. *reesei* is typically grown on either cellulose containing media (e.g. inexpensive mixture of soluble and/or insoluble sugars such as a cellulosic material Solca floc, paper mill waste streams) or lactose/milk whey at concentrations between 1 and 8 % (w/v) and a rich organic nitrogen source for a period up to 4 days and a pH between 3 and 6. Alternatively, T. reesei can be grown in solid-state fermentation, which makes use of the insoluble cellulosic materials to produce a solid phase which is wettened with soluble accessory nutrients, on which the fungal cell grows.

In the method of the present invention fungi of the family Hypocreaceae, in particular Trichoderma reesei cells, are used comprising the nucleic acid sequence having SEQ ID No. 3.

A further aspect of the present invention relates to a method for producing a recombinant protein in a fungal cell of the family Hypocreaceae comprising the steps of:
a) providing a wild type fungal cell of the family Hypocreaceae,
b) genetically modifying said wild type fungal cell by replacing the promoter of the gene encoding the regulatory protein VEL1 by a promoter capable to express the regulatory protein VEL1 in a higher amount compared to the unmodified wild type fungal cell or introducing additional copies of the vel1 gene into the genome or by introducing a vector according to the present invention,
c) introducing into said genetically modified fungal cell a nucleic acid molecule comprising at least one promoter of a cellulase gene, hemicellulase gene, carbohydrate esterase gene and/or gene of other homo- and heteropolysaccharide degrading enzymes such as chitinases operably linked to a heterologous coding region encoding the recombinant protein,
d) cultivating the genetically modified fungal cell of the family Hypocreaceae of step c) in a cultivation medium, and
e) isolating the recombinant protein produced by said fungal cell.

As already discussed the genetically modified fungal cell can also be used for the production of recombinant proteins and polypeptides.

The present invention is further illustrated by the following figures and examples, however, without being restricted thereto.
Fig. 1 shows biomass dry weight during growth on lactose by the parent strain QM 9414 (boxed) and a random selection of 5 strains bearing the tef1:vel1 gene construct.
Fig. 2 shows cellulase overproduction by T. reesei QM 9414 and the two best tef1:vel1 transformant strains. Three replicas were performed for every strain, and are given in vertical order. Activity was assayed using p-nitrophenyl-ß-D-cellobioside (Uzbas F, et al. Appl Microbiol Biotechnol 93(2012):1601-8).
Fig. 3 shows results from an SDS-PAGE using 10 µl of extracellular culture filtrate from QM 9414 and *lae1OE* after 72 and 96 h of cultivation under the same conditions as used for Fig. 1 and 2 (a, b, and c indicate biological triplicates).

### Embodiments:

Preferably, the present invention is defined as in the following embodiments:
1. A genetically modified fungal cell of the family Hypocreaceae capable to express the regulatory protein VEL1 in a higher amount compared to the unmodified wild type fungal cell.
2. Genetically modified fungal cell according to embodiment 1, wherein the naturally occurring gene encoding the VEL1 protein is operably linked to a recombinant promoter capable to induce the increased expression of the VEL1 protein compared to the unmodified wild type fungal cell.
3. Genetically modified fungal cell according to embodiment 1 or 2, wherein the fungal cell comprises an expression vector carrying the gene encoding the VEL1 protein operably linked to a promoter.
4. Genetically modified fungal cell according to embodiment 2 or 3, wherein the promoter is selected from the group consisting of *tef1* promoter, *gpd1* promoter, *pki1* promoter, enol promoter, *pgk1* promoter and *cdna1* promoter.
5. Genetically modified fungal cell according to any one of embodiments 1 to 4, wherein the fungal cell is a cell of the genus Trichoderma or Hypocrea, preferably Trichoderma reesei.
6. Genetically modified fungal cell according to any one of embodiments 1 to 5, wherein said genetically modified fungal cell is capable to express at least 20%, preferably at least 50%, more preferably at least 100%, more of the regulatory protein Vel1 compared to the unmodified wild type fungal cell.
7. Genetically modified fungal cell according to any one of embodiments 1 to 6, wherein said genetically modified fungal cell comprises a nucleic acid molecule comprising at least one promoter of a cellulase gene, hemicellulase gene, carbohydrate esterase gene and/or other genes of homo- and heteropolysaccharide degrading enzymes, preferably of a chitinase gene, operably linked to a heterologous coding region.
8. Genetically modified fungal cell according to embodiment 7, wherein the heterologous coding region encodes a protein of interest.
9. Genetically modified fungal cell of embodiment 7 or 8, wherein the at least one promoter of a cellulase gene, hemicellulase gene, carbohydrate esterase gene and/or gene of other homo- and heteropolysaccharide degrading enzymes such as chitinase is selected from the group consisting of endo-ß-1,4-glucanase genes egl12 or egl5, xylanase gene xyn2, the GH16 endo-ß-1,3/1,4-glucanase, the GH27 α-galactosidase gene, the cellobiohydrolae genes cbh1 and cbh2, the GH43 α-arabinofuranosidase gene, the endochitinase gene, and the acetyl-xylan esterase gene axel.
10. A vector comprising a nucleic acid molecule encoding a regulatory protein VEL1 of the family Hypocreaceae, preferably of the genus Trichoderma or Hypocrea, having at least 80% identity to SEQ ID No. 1 and a promoter operably linked to said nucleic acid molecule.
11. Vector according to embodiment 10, wherein the promoter is not the native *vel1* promoter.
12. Vector according to embodiment 10 or 11, wherein the promoter is selected from the group consisting of *tef1* promoter, *gpd1* promoter, *pki1* promoter, *eno1* promoter, *pgk1* promoter and *cdna1* promoter.
13. Cell of the family Hypocreaceae, preferably of the genus Trichoderma or Hypocrea, comprising a vector according to any one of embodiments 10 to 12.
14. A method for producing cellulases, hemicellulases, carbohydrate esterases and/or other homo- and heteropolysaccharide degrading enzymes in a fungal cell of the family Hypocreaceae, preferably of the genus Trichoderma or Hypocrea, comprising the steps of:
   a) providing a wild type fungal cell of the family Hypocreaceae or a mutated variant thereof,
   b) genetically modifying said wild type fungal cell or the mutated variant thereof by replacing the promoter of the gene encoding the regulatory protein VEL1 by a promoter capable to express the regulatory protein VEL1 in a higher amount compared to the unmodified wild type fungal cell or introducing additional copies of the vel1 gene into the genome or by introducing a vector according to any one of embodiments 10 to 12,
   c) cultivating the genetically modified fungal cell of the family Hypocreaceae of step b) in a cultivation medium, and
   d) isolating the cellulases, hemicellulases, carbohydrate esterases and/or other homo- and heteropolysaccharide degrading enzymes produced by said fungal cell.
15. A method for producing a recombinant protein in a fungal cell of the family Hypocreaceae, preferably of the genus Trichoderma or Hypocrea, comprising the steps of:
   a) providing a wild type fungal cell of the family Hypocreaceae,
   b) genetically modifying said wild type fungal cell by replacing the promoter of the gene encoding the regulatory protein VEL1 by a promoter capable to express the regulatory protein VEL1 in a higher amount compared to the unmodified wild type fungal cell or introducing additional copies of the vel1 gene into the genome or by introducing a vector according to any one of embodiments 10 to 12,
   c) introducing into said genetically modified fungal cell a nucleic acid molecule comprising at least one promoter of a cellulase gene, hemicellulase gene, carbohydrate esterase gene and/or gene of other homo- and heteropolysaccharide degrading enzymes operably linked to a heterologous coding region encoding the recombinant protein,
   d) cultivating the genetically modified fungal cell of the family Hypocreaceae of step c) in a cultivation medium, and
   e) isolating the recombinant protein produced by said fungal cell.
16. Method according to embodiment 15, wherein the heterologous coding region encodes a protein of interest.
17. Method according to embodiment 15 or 16, wherein the at least one promoter of a cellulase gene, hemicellulase gene, carbohydrate esterase gene and/or gene of other homo- and heteropolysaccharide degrading enzymes is selected from the group consisting ofendo-ß-1,4-glucanase genes, xylanase genes, GH16 endo-ß-1,3/1,4-glucanase genes, GH27 α-galactosidase genes, cellobiohydrolase genes, GH43 α-arabinofuranosidase genes, endochitinase genes, and acetyl-xylan esterase genes.
18. Method according to any one of embodiments 15 to 17, wherein the cultivation medium in which the genetically modified fungal cell is cultivated comprises cellulosic or hemicellulosic carbohydrates and/or lactose, and other components that lead to the induction of cellulase, hemicellulases and other heteropolysaccharide hydrolyzing enzymes.

### EXAMPLE:

The aim of the following examples is to show that overexpression of the global developmental regulator VEL1 of T. reesei significantly enhances cellulase and hemicellulase production and this manipulation can thus be used for strain improvement.

### Material and Methods

### Identification of T. reesei vel1

For the identification of the T. reesei VEL1 orthologue the LaeA proteins from A. nidulans and A. fumigatus were used to retrieve the respective orthologue from T. reesei by a BLASTP ysearch at http://genome.jgi-psf.org/Trire2/Trire2.home.html.

This led to the identification of Trire2:122284/estExt_fgenesh5_pg.C_110199 as the T. reesei orthologue of Aspergillus VeA. The deduced amino acid sequence is SEQ ID No. 1:
SEQ ID No. 1: amino acid sequence of T. reesei VEL1

MATPSSVASSSSRDQVQRIHRVTRENRHLWYQLTVLQQPERARACGSGMKANSDRRPVDPPPVV
ELRIIEGPSVEEGKDITFDYNANFFLYASLEQARTIAHGRVQNGATNNPPILTGVPASGMAYLD
RPTEAGYFIFPDLSVRHEGYFRLSFSLYETTKEPKDFDLEPADSDLPPGVDWRMEIKTQPFNVF
SAKKFPGLMESTSLSKTVADQGCRVRIRRDVRMRKRDGKGSGFDRRGEEEYSRRRTVTPAPAED
PNLRARSVSNASEHRGPYMPQEPPRRPSAAESYHAPPPLPPPPPSSYDAPPPAARPGHLGFGGD
HNMPPQYGAPAARPYGHPQSAPIPPATPTGPYPTSSAAPSPYAKQDHHHQQYSYNSRPPAPSA
SPAPPMKHAMYDNRPSEPYVPHSSPSVYASTERRPSYANYSAPAPYSTPASQPTYSTPASQPTY
STPASQPTYSAPPPAPYSAPAPPPPRPSMSQSSLAPLKIASLVSPLPPIEAQTEPLPPPPMLST
GGKRKHDHVFSQNHKPLYNGQRQLDAHYGHGYRGLTPEPDQGLYSRADGQIGVVTFNQYQV

The nucleotide sequence of the vel1 locus is SEQ ID No. 2:
SEQ ID No. 2: nucleotide sequence of the genomic vel1 locus. The coding nucleotides are underlined. 
ATGGCGACGCCTTCCTCCGTGGCCTCGTCCAGCTCGCGGACCAGGTCCAGCGCATCCATCGCG
TGACGAGAGAGAACCGTCACCTTTGGTACCAGTTGACGGTTCTACAGCAGCCGGAGCGAGCCCG
TGCATGCGGTTCAGGCATGAAAGGTGTGTTGGCCACTCGACGGAACCAAGTGAACAGGCGATGG
CGCGTTGCAGGCCCCCCCCTTTGCTGACCCTCTCACAGCGAACAGTGACCGACGACCAGTTGAT
CCGCCGCCTGTTGTCGAGCTCCGTATCATCGAAGGTCCTTCTGTCGAGGAGGGCAAGGACATTA
 
CTTTCGACTACAATGCCAACTTCTTTCTTTATGCCAGTCTGGAGCAGGCTCGCACGATTGCTCA
CGGCCGCGTCCAAAACGGCGCCACCAACAATCCTCCCATCCTGACTGGTGTTCCTGCATCGGGC
ATGGCATACCTCGACCGGCCAACCGAGGCTGGATATTTCATCTTCCCCGACCTGTCCGTCCGAC
ACGAAGGCTACTTCCGCCTGTCCTTTAGCTTGTACGAGACGACCAAGGAGCCCAAGGACTTTGA
CCTAGAGCCGGCCGATTCCGACCTTCCCCCTGGGGTGGACTGGAGGATGGAGATCAAGACGCAG
CCTTTCAACGTCTTCAGCGCCAAGAAGTTCCCTGGACTCATGGAGAGTACCTCGCTCAGCAAGA
CCGTTGCCGACCAGGGCTGCCGAGTTCGAATCCGCCGCGACGTTCGCATGAGGAAGCGCGACGG
CAAGGGCAGCGGCTTTGACCGACGCGGTGAGGAGGAGTACTCTCGCCGCCGAACCGTGACGCCA
GCTCCCGCCGAGGATCCCAACCTGCGAGCCAGATCCGTCAGCAACGCGAGCGAGCACCGCGGGC
CGTACATGCCGCAGGAGCCGCCACGGCGACCATCCGCTGCCGAGAGCTACCATGCCCCTCCCCC
ACTGCCTCCTCCGCCGCCTTCGTCGTACGACGCTCCTCCGCCTGCCGCTCGTCCCGGACATCTG
GGCTTTGGCGGCGATCACAACATGCCGCCGCAATATGGCGCACCCGCCGCTCGGCCATACGGCC
ACCCGCAGAGCGCACCGATACCTCCTGCGACACCCACCGGACCCTACCCGACATCGTCCGCGGC
GCCGTCTCCGTATGCCAAGCAGGATCATCATCATCAGCAGCAGTACAGCTACAACTCCCGCCCT
CCGGCGCCCAGTGCTTCCCCAGCGCCGCCGATGAAGCACGCCATGTACGACAACCGGCCATCCG
AGCCTTATGTGCCTCACTCCTCGCCTTCCGTATATGCGTCGACCGAGAGGCGACCTTCTTATGC
CAACTACTCTGCTCCTGCGCCCTATTCTACTCCCGCCTCCCAGCCGACCTACTCTACTCCCGCC
TCCCAGCCGACCTACTCTACTCCCGCCTCTCAGCCGACCTACTCGGCTCCCCCGCCAGCGCCAT
ACTCGGCTCCGGCGCCTCCTCCTCCTAGGCCGTCGATGTCTCAGTCGTCTCTGGCGCCGCTCAA
GATTGCGTCCCTGGTGTCTCCCCTGCCGCCCATTGAGGCTCAGACCGAGCCTCTTCCTCCGCCT
CCTATGCTGTCGACCGGTGGCAAGAGAAAACATGACCACGTCTTCTCCCAGAACCACAAGCCGC
TGTACAACGGCCAGCGCCAGCTTGACGCGCACTATGGCCACGGATATCGCGGGCTCACCCCCGA
GCCCGACCAGGGCCTATACTCTCGTGCGGACGGTCAAATCGGCGTTGTCACCTTCAACCAATAC
CAGGTGTAAGGCCATTGGACGAGTGTTTGTTTGACTCGGACCCTTGGGTGACACGGATTCACTG
GAGTATATTATCTGCATTTTTTGATGTGTGATAGATTTCTCATGG
SEQ ID No. 3: nucleotide sequence of the tef1:vel1 construct (5'→3').

GGACAGAATGTACAGTACTATACTACCTACCTTAGATGCCATCACCCCTTCGCAGGCCATGGAA
TTTCTGGCAAGTCGCACCCTTCAGCCATGCCTGTCCGCCATCATCATACCAGATCGTGTCGTGG
CGGGCACTTGGGAAGGACGAGCTCCAACCGCCGCCGCTGATTGGCCTGATCCGCCACAGCCACT
GGCGCTCGCGGCGAGGAAAAAAAAAAATCTTGCGGGTGTGCAGCAGCATTGAGTGGAAAGCGGG
AGCCGCTGCACGACGCTGATTCGTCCGGGCTGCCTCGTTAGGGCTAGACCAGCAGGCGGGCGCC
GCTTATTTTGCCCCTCCTCAGGCTTTTTTCACAAGCCCCGTTTTGCTGCCCCGCGCGTGGCAG
AAAAAAAAAATACAGCACGAGCCACTGGCACTTGCACCCCTCCACCCCTCCGCCGACCACCTTT
TAAATCTCCTCCTCCCACCATCTGCTTTCGAAATTTTCTCTCTTCTGCTCTTCGTCTCGCATAC
CCGGTTCAAGCATCCGATCTGCGAATTTGTATGTGCTGCCTCTCCCTCTGACCTTCTGGTCTGG
TGATACCATCCTCCCTCAGTTTGGATCATCGCCTTATTCTTCTTCCCTCTTCTGCATCTGCTTC
CTGCTCGTTTGAGGAACATCGCCAGCTGACTCTGCTTGCCTCGCAGCGATCTAGTCAAGAACAA

CACAGCTCTCACGCTACATCACACAAACCGTCATCGATGGCGACGCCTTCCTCCGTGGCCTCGT
CCAGCTCGCGGGACCAGGTCCAGCGCATCCATCGCGTGACGAGAGAGAACCGTCACCTTTGGTA
CCAGTTGACGGTTCTACAGCAGCCGGAGCGAGCCCGTGCATGCGGTTCAGGCATGAAAGGTGTG
TTGGCCACTCGACGGAACCAAGTGAACAGGCGATGGCGCGTTGCAGGCCCCCCCTTTGCTGAC
CCTCTCACAGCGAACAGTGACCGACGACCAGTTGATCCGCCGCCTGTTGTCGAGCTCCGTATCA
TCGAAGGTCCTTCTGTCGAGGAGGGCAAGGACATTACTTTCGACTACAATGCCAACTTCTTTCT
TTATGCCAGTCTGGAGCAGGCTCGCACGATTGCTCACGGCCGCGTCCAAAACGGCGCCACCAAC
AATCCTCCCATCCTGACTGGTGTTCCTGCATCGGGCATGGCATACCTCGACCGGCCAACCGAGG
CTGGATATTTCATCTTCCCCGACCTGTCCGTCCGACACGAAGGCTACTTCCGCCTGTCCTTTAG
CTTGTACGAGACGACCAAGGAGCCCAAGGACTTTGACCTAGAGCCGGCCGATTCCGACCTTCCC
CCTGGGGTGGACTGGAGGATGGAGATCAAGACGCAGCCTTTCAACGTCTTCAGCGCCAAGAAGT
TCCCTGGACTCATGGAGAGTACCTCGCTCAGCAAGACCGTTGCCGACCAGGGCTGCCGAGTTCG
AATCCGCCGCGACGTTCGCATGAGGAAGCGCGACGGCAAGGGCAGCGGCTTTGACCGACGCGGT
GAGGAGGAGTACTCTCGCCGCCGAACCGTGACGCCAGCTCCCGCCGAGGATCCCAACCTGCGAG
CCAGATCCGTCAGCAACGCGAGCGAGCACCGCGGGCCGTACATGCCGCAGGAGCCGCCACGGCG
ACCATCCGCTGCCGAGAGCTACCATGCCCCTCCCCCACTGCCTCCTCCGCCGCCTTCGTCGTAC
GACGCTCCTCCGCCTGCCGCTCGTCCCGGACATCTGGGCTTTGGCGGCGATCACAACATGCCGC
CGCAATATGGCGCACCCGCCGCTCGGCCATACGGCCACCCGCAGAGCGCACCGATACCTCCTGC
GACACCCACCGGACCCTACCCGACATCGTCCGCGGCGCCGTCTCCGTATGCCAAGCAGGATCAT
CATCATCAGCAGCAGTACAGCTACAACTCCCGCCCTCCGGCGCCCAGTGCTTCCCCAGCGCCGC
CGATGAAGCACGCCATGTACGACAACCGGCCATCCGAGCCTTATGTGCCTCACTCCTCGCCTTC
CGTATATGCGTCGACCGAGAGGCGACCTTCTTATGCCAACTACTCTGCTCCTGCGCCCTATTCT
ACTCCCGCCTCCCAGCCGACCTACTCTACTCCCGCCTCCCAGCCGACCTACTCTACTCCCGCCT
CTCAGCCGACCTACTCGGCTCCCCCGCCAGCGCCATACTCGGCTCCGGCGCCTCCTCCTCCTAG
GCCGTCGATGTCTCAGTCGTCTCTGGCGCCGCTCAAGATTGCGTCCCTGGTGTCTCCCCTGCCG
CCCATTGAGGCTCAGACCGAGCCTCTTCCTCCGCCTCCTATGCTGTCGACCGGTGGCAAGAGAA
AACATGACCACGTCTTCTCCCAGAACCACAAGCCGCTGTACAACGGCCAGCGCCAGCTTGACGC
GCACTATGGCCACGGATATCGCGGGCTCACCCCCGAGCCCGACCAGGGCCTATACTCTCGTGCG
GACGGTCAAATCGGCGTTGTCACCTTCAACCAATACCAGGTGTAAGGCCATTGGACGAGTGTTT
GTTTGACTCGGACCCTTGGGTGACACGGATTCACTGGAGTATATTATCTGCATTTTTGATGTG
TGATAGATTTCTCATGGGCGTTTTGGGACCAGTACCCTTGTTCAAAATACCTCTTTTGTTTTCT
GTTGGAAGAGGGAAATACGAAGGAACTTTTGCGGGTATTAACGGAGTTTGGGAACACATGACA
ATAGTTTGAGGGAAATTTCTTTCATTCATCTTTCTCTTTTCCTTATCCTTTCTTTGTCTGTTAA
AGTGAGATGGTTCAACGGAACACAGCGACTTCATTCCAACTCTCCCCTTTGCATCTCATTCTTC
TGTTGTCTTGCCATTTTTCGATATCTTTTATCCGTGGGCACCCTTTATGCGTGGAATGCTAATG
AGAGGGACCTGGGATATGGGGAGTTTTCTTTCTCTTGCCTTTTTTTTTTCTTCCTGAAAGCTTT
GGGGTACTA

### Strain construction

T. reesei QM 9414 (ATCC 26921) was used in these examples.

Escherichia coli JM109 (Promega) was used for plasmid construction and amplification.

A 2,275-bp vel1 PCR fragment including the coding and terminator region was amplified with the oligonucleotides Fw_ Ptef1:vell-Cla1 (GCTATCGATGGCGACGCCTTCCTCCGT) (SEQ ID No. 4) and tef1:vel1-HindIII (TAGTACCCCAAAGCTTTCAGGA) (SEQ ID No. 5) and then inserted downstream of the tef1 promoter region (Genbank accession number Z23012.1 (see SEQ ID No. 3) into the ClaI/HindIII sites of pLH1hphtef1 resulting in vector pRKA-OE122284hph, which contains the E. coli hygromycin B phosphotransferase (hph) under T. reesei expression signals as selection marker (Akel et al., Eukaryot Cell 8(2009): 1837-1844). All vectors constructed were verified by sequencing.

Protoplast preparation and DNA mediated transformation was described (Guangtao et al., J Biotechnol 139(2009): 146-151). The strains were purified twice for mitotic stability, and integration of the expression cassettes was verified by PCR analysis.

For genomic DNA extraction, fungal mycelia were harvested by filtration, washed with distilled cold water, frozen and ground under liquid nitrogen. Purification kits (Wizard Genomic DNA Purification Kit, and PureYield Plasmid Midiprep System from Promega) were used according to the manufacturer's protocol. Standard methods were used for electrophoresis, blotting and hybridization of nucleic acids.

### Cellulase production

For cellulase production, T. reesei was grown in Mandels-Andreotti medium using Avicel cellulose, lactose, oat spelts xylan or glycerol as a carbon source (1 %, w/v) as stated at the respective results.

### Biochemical assays

Cellulase enzyme activities were determined using carboxymethylcellulose (1 %, w/v) and p-nitrophenyl-ß-D-cellobioside (Uzbaz et al. Appl Microbiol Biotechnol 93(2012): 1601-1608). Xylanases, ß-xylosidase and ß-glucosidase activities were determined as described previously (Kubicek et al. 1988 J. Gen. Microbiol. 134: 1215 - 1222).

### SDS-PAGE

SDS-PAGE was performed as described by Ausubel et al. 1999 (Short Protocols in Molecular Biology : A Compendium of Methods from Current Protocols in Molecular Biology , Wiley & Sons).

### qPCR

DNase treated (DNase I, RNase free; Fermentas) RNA (5µg) was reverse transcribed with the RevertAid™ First Strand cDNA Kit (Fermentas) according to the manufacturer's protocol with a combination of oligo-dT and random hexamer primers. All real-time RT-PCR experiments were performed on a Bio-Rad iCycler IQ. For the reaction the IQ SYBR Green Supermix (Bio-Rad) was prepared for 25 µl assays with standard MgCl₂ concentration (3 mM) and primers listed in Table 1 with final concentration of 100 nM each.

**Table 1. Oligonucleotides used in quantitative real-time PCR.**

| **Gene ID** | **Oligonucleotide** | **Sequence** | **Amplification Efficiency [%]** | **R² value** |
|---|---|---|---|---|
| 123902/tef1 | tef1 qPCR fw | 5'-CCACATTGCCTGCAAGTTCGC-3' | 91 | 0,998 |
| | tef1 qPCR rv | 5'-GTCGGTGAAAGCCTCAACGCAC-3' | | |
| 72567/cel6a | cbh2 qPCR fw | 5'-ACTACAACGGGTGGAACATTAC-3' | 106 | 0,999 |
| | cbh2 qPCR rv | 5'-CGTGGATGTACAGCTTCTCG-3' | | |
| 123989/cel7a | cbhl qPCR fw | 5'-CCGAGCTTGGTAGTTACTCTG-3' | 101 | 0,999 |
| | cbh1 qPCR rv | 5'-GGTAGCCTTCTTGAACTGAGT-3' | | |

All assays were carried out in 96-well plates. Determination of the PCR efficiency was performed using triplicate reactions from a dilution series of cDNA (1; 0.1; 0.01; 0.001). Amplification efficiency was then calculated from the given slopes in the IQ5 Optical system Software v2.0. Expression ratios were calculated using REST© Software (Pfaffl et al., Nucleic Acids Res 30(2002): e36). All samples were analyzed in at least two independent experiments with three replicates in each run.

Basic statistical methods such as multiple regression analysis and analysis of variance (ANOVA) as well as multivariate exploratory techniques (cluster and factor analyses) were performed using STATISTICA 6.1 (StatSoft, Inc.) data analysis software system.

### Results:

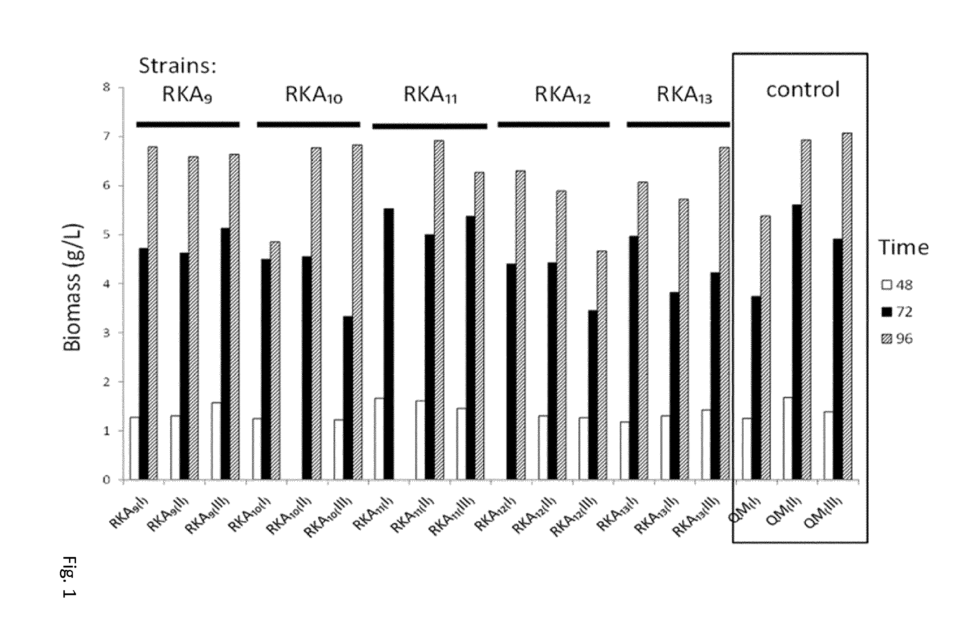

The vel1 ORF was fused to the 5'-upstream sequences of the tef1 (translation elongation factor 1α-encoding) gene, which was expected to result in high constitutive expression of vell.T. reesei strains that contained either one or more copies of the tef1:vel1 construct integrated ectopically in the genome were examined for their growth: all transformants exhibited comparable biomass formation until 96 hrs of cultivation (Fig. 1).

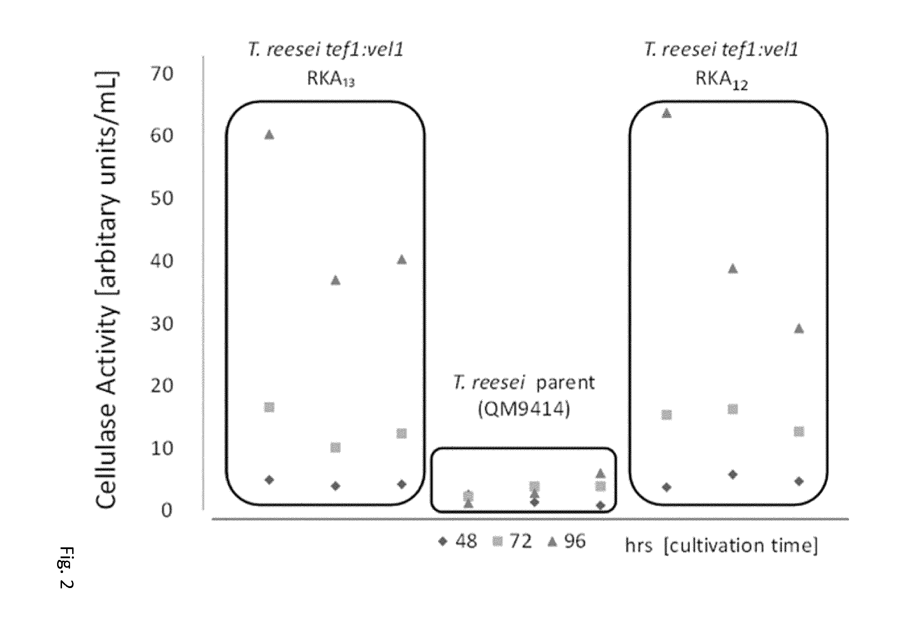

The two best strains bearing the tef1:lae1 copies exhibited up to 20-fold increased formation of cellulase formation on lactose as a carbon source (Fig. 2). The expression of cel7A and cel6A by qPCR (48 hrs) was also quantified and it could be shown that they were 3-fold increased (p < 0.001).

In order to test whether these findings also apply to other enzymes commonly secreted by T. reesei under cellulase inducing conditions, the formation of xylanase-, ß-xylosidase- and ß-glucosidase activity on lactose, xylan and cellulose as a carbon source was tested. The respective data are shown in Table 2.

**Table 2. Formation of cellulase and hemicellulase enzyme activities by T. reesei QM 9414 (parent strain) and the vel1OE strain***

| | | | **QM 9414** | |
|---|---|---|---|---|
| | | 72 h | 96 h | 120 h |
| ß-xylosidase | cellulose | 0,215 [± 0,07] | 0,145 [± 0,07] | |
| | xylan | 0,455 [± 0,05] | 0,580 [± 0,09] | |
| ß-glucosidase | cellulose | 0,47 [± 0,035] | 0,66 [± 0,05] | |
| | lactose | 0,273 [± 0,04] | | |
| xylanase | cellulose | | 0,36 [± 0,03] | 0,37 [± 0,06] |
| | xylan | | 0,33 [± 0,03] | 0,28 [± 0,09] |
| cellulase * | cellulose | 0,04 [± 0,02] | 0,12 [± 0,045] | 0,13 [± 0,05] |

| | | | ***vel1OE*** | |
|---|---|---|---|---|
| | | 72 h | 96 h | 120 h |
| ß-xylosidase | cellulose | 0,53 [± 0,12] | 0,334 [± 0,08] | |
| | xylan | 0,72 [± 0,08] | 1,30 [± 0,10] | |
| ß-glucosidase | cellulose | 0,57 [± 0,04] | 0,78 [± 0,07] | |
| | lactose | 0,630 [± 0,08] | | |
| xylanase | cellulose | | 0,487 [± 0,06] | 0,585 [± 0,075] |
| | xylan | | 0,430 [± 0,07] | 0,530 [± 0,045] |
| cellulase * | cellulose | 0,130 [± 0,03] | 0,165 [± 0,04] | 0,190 [± 0,04] |

Values are means of 3-6 biological replicates and 2-4 independent measurements. Numbers in brackets indicate standard deviations.

* measured by the ability to hydrolyse carboxymethylcellulose

All of the enzymes tested indeed exhibited higher activities in the vel1 overexpressing strains.

Also the formation of cellulases on cellulose was tested, using the carboxymethyl-cellulose hydrolysis assay (Uzbaz et al. Appl Microbiol Biotechnol 93(2012): 1601-1608), and confirmed increased activities in the tef1:vel1 strain (Table 2).

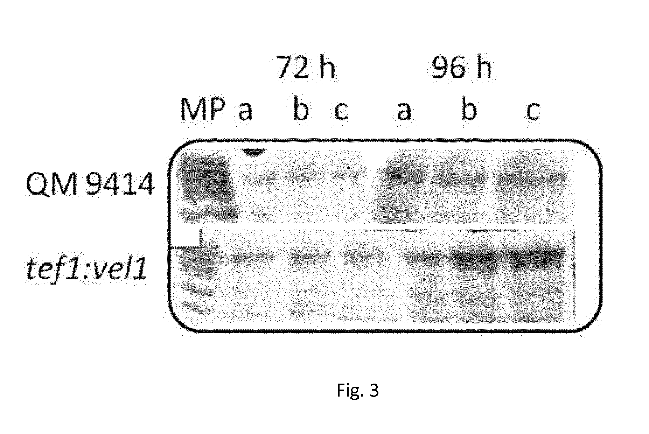

Finally, the concentration of extracellular proteins was compared by SDS-PAGE (Fig. 3), using identical volumens of the cultutre supernatant from QM 9414 and *vel1OE*. The results document the higher protein secretion in the strain overexpressing VEL1.

## Claims

1. A genetically modified fungal cell of the family Hypocreaceae capable to express the regulatory protein VEL1 in a higher amount compared to the unmodified wild type fungal cell.

2. Genetically modified fungal cell according to claim 1, wherein the naturally occurring gene encoding the VEL1 protein is operably linked to a recombinant promoter capable to induce the increased expression of the VEL1 protein compared to the unmodified wild type fungal cell.

3. Genetically modified fungal cell according to claim 1 or 2, wherein the fungal cell comprises an expression vector carrying the gene encoding the VEL1 protein operably linked to a promoter.

4. Genetically modified fungal cell according to claim 2 or 3, wherein the promoter is selected from the group consisting of *tef1* promoter, *gpd1* promoter, *pki1* promoter, *eno1* promoter, *pgk1* promoter and *cdna1* promoter.

5. Genetically modified fungal cell according to any one of claims 1 to 4, wherein said genetically modified fungal cell comprises a nucleic acid molecule comprising at least one promoter of a cellulase gene, hemicellulase gene, carbohydrate esterase gene and/or other genes of homo- and heteropolysaccharide degrading enzymes, preferably of a chitinase gene, operably linked to a heterologous coding region.

6. Genetically modified fungal cell according to claim 5, wherein the heterologous coding region encodes a protein of interest.

7. Genetically modified fungal cell of claim 5 or 6, wherein the at least one promoter of a cellulase gene, hemicellulase gene, carbohydrate esterase gene and/or gene of other homo- and heteropolysaccharide degrading enzymes such as chitinase is selected from the group consisting of endo-ß-1,4-glucanase genes egl12 or egl5, xylanase gene xyn2, the GH16 endo-ß-1,3/1,4-glucanase, the GH27 α-galactosidase gene, the cellobiohydrolae genes cbh1 and cbh2, the GH43 α-arabinofuranosidase gene, the endochitinase gene, and the acetyl-xylan esterase gene axel.

8. A vector comprising a nucleic acid molecule encoding a regulatory protein VEL1 of the family Hypocreaceae, preferably of the genus Trichoderma or Hypocrea, having at least 80% identity to SEQ ID No. 1 and a promoter operably linked to said nucleic acid molecule.

9. Vector according to claim 8, wherein the promoter is selected from the group consisting of *tef1* promoter, *gpd1* promoter, *pki1* promoter, *eno1* promoter, *pgk1* promoter and *cdna1* promoter.

10. Cell of the family Hypocreaceae, preferably of the genus Trichoderma or Hypocrea, comprising a vector according to any one of claims 8 to 9.

11. A method for producing cellulases, hemicellulases, carbohydrate esterases and/or other homo- and heteropolysaccharide degrading enzymes in a fungal cell of the family Hypocreaceae, preferably of the genus Trichoderma or Hypocrea, comprising the steps of:
a) providing a wild type fungal cell of the family Hypocreaceae or a mutated variant thereof,
b) genetically modifying said wild type fungal cell or the mutated variant thereof by replacing the promoter of the gene encoding the regulatory protein VEL1 by a promoter capable to express the regulatory protein VEL1 in a higher amount compared to the unmodified wild type fungal cell or introducing additional copies of the vel1 gene into the genome or by introducing a vector according to any one of claims 8 to 9,
c) cultivating the genetically modified fungal cell of the family Hypocreaceae of step b) in a cultivation medium, and
d) isolating the cellulases, hemicellulases, carbohydrate esterases and/or other homo- and heteropolysaccharide degrading enzymes produced by said fungal cell.

12. A method for producing a recombinant protein in a fungal cell of the family Hypocreaceae, preferably of the genus Trichoderma or Hypocrea, comprising the steps of:
a) providing a wild type fungal cell of the family Hypocreaceae,
b) genetically modifying said wild type fungal cell by replacing the promoter of the gene encoding the regulatory protein VEL1 by a promoter capable to express the regulatory protein VEL1 in a higher amount compared to the unmodified wild type fungal cell or introducing additional copies of the vel1 gene into the genome or by introducing a vector according to any one of claims 8 to 9,
c) introducing into said genetically modified fungal cell a nucleic acid molecule comprising at least one promoter of a cellulase gene, hemicellulase gene, carbohydrate esterase gene and/or gene of other homo- and heteropolysaccharide degrading enzymes operably linked to a heterologous coding region encoding the recombinant protein,
d) cultivating the genetically modified fungal cell of the family Hypocreaceae of step c) in a cultivation medium, and
e) isolating the recombinant protein produced by said fungal cell.

13. Method according to claim 12, wherein the heterologous coding region encodes a protein of interest.

14. Method according to claim 12 or 13, wherein the at least one promoter of a cellulase gene, hemicellulase gene, carbohydrate esterase gene and/or gene of other homo- and heteropolysaccharide degrading enzymes is selected from the group consisting ofendo-ß-1,4-glucanase genes, xylanase genes, GH16 endo-ß-1,3/1,4-glucanase genes, GH27 α-galactosidase genes, cellobiohydrolase genes, GH43 α-arabinofuranosidase genes, endochitinase genes, and acetyl-xylan esterase genes.

15. Method according to any one of claims 12 to 14, wherein the cultivation medium in which the genetically modified fungal cell is cultivated comprises cellulosic or hemicellulosic carbohydrates and/or lactose, and other components that lead to the induction of cellulase, hemicellulases and other heteropolysaccharide hydrolyzing enzymes.
